# EUROPEAN PATENT SPECIFICATION

(11) **EP 3 682 857 B1**
(45) Date of publication and mention of the grant of the patent: **02.08.2023**
(21) Application number: 17929383.2
(22) Date of filing: 20.10.2017
(51) Int. Cl.: A61F 13/15, B02C 23/02, B65H 19/18, D21B 1/06, D21C 9/00

(54) **ABSORBENT BODY PRODUCTION METHOD AND ABSORBENT BODY PRODUCTION DEVICE**
VERFAHREN ZUR HERSTELLUNG EINES ABSORBIERENDEN KÖRPERS UND VORRICHTUNG ZUR HERSTELLUNG EINES ABSORBIERENDEN KÖRPERS
PROCÉDÉ DE PRODUCTION D'UN CORPS ABSORBANT ET DISPOSITIF ASSOCIÉ

(43) Date of publication of application: 22.07.2020
(73) Proprietor: Unicharm Corporation, Shikokuchuo-shi, Ehime 799-0111 (JP)
(72) Inventor: SHINOMORI, Youji, Kanonji-shi Kagawa 769-1602 (JP)
(74) Representative: Staeger & Sperling Partnerschaftsgesellschaft mbB
(86) International application number: PCT/JP2017/038006
(87) International publication number: WO 2019/077741

(56) References cited:
- WO-A1-2014/087902
- JP-A- H09 278 237
- JP-A- 2011 152 351
- US-A1- 2013 037 635
- US-A1- 2017 233 210

## Description

### [Technical Field]

The present invention relates to a method and an apparatus for manufacturing an absorbent body.

### [Background Art]

An absorbent article includes an absorbent body that absorbs a liquid such as an excreted fluid. Fibrous pulp obtained by pulverizing a pulp sheet with a pulverizer is used as the material making up the absorbent body.

### [Citation List]

### [Patent Literature]

[PTL 1] Japanese Patent Application Publication No. 2011-152351. Further prior art in this technical field ist disclosed in document WO 2014/087902 A1.

### [Summary of Invention]

### [Technical Problem]

Ordinary fibrous pulp is produced by a pulp sheet being fed into a pulverizer, and the leading end of the fed pulp sheet being shredded by pulverizing blades of the pulverizer. One example of an ordinary method for pulverizing the trailing end (terminal end) of a pulp sheet in this production method is a method in which a preceding pulp sheet and a new following pulp sheet are each cut along the width direction and connected to each other using a joint material or the like, and then the preceding pulp sheet and the following pulp sheet are pulverized in this order.

However, with this method, problems occur in fibrous pulp production, and there has been a problem in terms of a decrease in the quality of the absorbent body.

The present invention was achieved in light of conventional problems such as that described above, and an aspect of the present invention is to suppress a decrease in the quality of the absorbent body.

### [Solution to Problem]

A main aspect of the present invention for achieving the above-described aspect is a method for manufacturing an absorbent body including:
pulverizing to produce a fibrous pulp that is to serve as a material of an absorbent body, by using a feed roller to feed a pulp sheet toward a pulverizer in a feeding direction while supporting the pulp sheet from one end to another end in a width direction of the pulp sheet, and pulverizing the fed pulp sheet by successively shredding a leading end of the pulp sheet with use of a plurality of moving pulverizing blades of the pulverizer;
cutting to cut a trailing end portion of a preceding pulp sheet and a leading end portion of a new following pulp sheet; and
joining to produce a jointed pulp sheet by joining the preceding pulp sheet and the following pulp sheet with use of a joint material,
in the pulverizing, the jointed pulp sheet fed with the feed roller being pulverized in order of first the preceding pulp sheet and then the following pulp sheet,
in the cutting, the trailing end portion of the preceding pulp sheet and the leading end portion of the new following pulp sheet being diagonally cut such that a diagonal cut line of the trailing end portion and a diagonal cut line of the leading end portion have the same inclination, and such that Condition 1 shown below is satisfied with respect to the inclination, and
in the joining, the preceding pulp sheet and the following pulp sheet being joined with use of the joint material in a state where the diagonal cut line of the trailing end portion and the diagonal cut line of the leading end portion have been brought together,
Condition 1: the length of the diagonal cut lines in the feeding direction > a shortest distance from a support position, at which the feed roller supports the pulp sheet, to a movement locus of blade tips of the pulverizing blades.

Features of the present invention other than the above will become clear by reading the description of the present specification with reference to the accompanying drawings.

### [Advantageous Effects of Invention]

According to the present invention, it is possible to suppress a decrease in the quality of an absorbent body.

### [Brief Description of the Drawings]

FIG. 1 is a conceptual side view of an apparatus 10 for manufacturing an absorbent body according to an embodiment (first embodiment).
FIG. 2 is a switch timing line chart showing the timing of switching between rollers that feed pulp sheets 1 to a pulverizer 30, specifically switching from a first feed roller 21 that is feeding a pulp sheet 1 to a second feed roller 22 that is stopped.
FIG. 3 is a conceptual side view of a jointed pulp sheet production device 50.
FIG. 4A is a diagram in which a new sheet roll 1r has been attached, FIG. 4B is a diagram in which the new sheet roll 1r is rotating, FIG. 4C is a diagram in which the leading end portion of the new sheet roll 1r has moved to a moving portion 51d, and FIG. 4D is a diagram in which the moving portion 51d is moving while holding a new following pulp sheet 1f.
FIG. 5A is a diagram showing cutting operations of a cutting device 53, and FIG. 5B is a conceptual top view and a conceptual side view of a jointed pulp sheet.
FIG. 6 is an enlarged view that shows the positional relationship between a feed roller 20 and pulverizing blades 31b.
FIG. 7 is an enlarged view of a moving portion 51d.
FIG. 8 is a flowchart showing jointed pulp sheet production control.
FIG. 9A is a diagram in which a new following pulp sheet 1f has been arranged in the jointed pulp sheet production device 50, FIG. 9B is a diagram in which the cutting device 53 is cutting a preceding pulp sheet 1p and the following pulp sheet 1f, FIG. 9C is a diagram in which a joining device 54 is producing a jointed pulp sheet, and FIG. 9D is a diagram showing the jointed pulp sheet production device 50 after having produced the jointed pulp sheet.
FIG. 10A is a diagram in which a jointed pulp sheet that has been cut along the width direction is being pulverized, and FIG. 10B is a diagram in which a jointed pulp sheet that has been cut diagonally is being pulverized.
FIG. 11 is a diagram showing a state of the pulp sheet 1 in the case where an adhesion region J of the pulp sheet 1 has separated in an apparatus 10 for manufacturing an absorbent body that does not include regulation members 55.
FIG. 12 is a conceptual side view of an apparatus 10 for manufacturing an absorbent body according to a second embodiment.

### [Description of Embodiments]

At least the following matters will become clear with the description of this specification and the attached drawings.

A method for manufacturing an absorbent body including:
pulverizing to produce a fibrous pulp that is to serve as a material of an absorbent body, by using a feed roller to feed a pulp sheet toward a pulverizer in a feeding direction while supporting the pulp sheet from one end to another end in a width direction of the pulp sheet, and pulverizing the fed pulp sheet by successively shredding a leading end of the pulp sheet with use of a plurality of moving pulverizing blades of the pulverizer;
cutting to cut a trailing end portion of a preceding pulp sheet and a leading end portion of a new following pulp sheet; and
joining to produce a jointed pulp sheet by joining the preceding pulp sheet and the following pulp sheet with use of a joint material,
in the pulverizing, the jointed pulp sheet fed with the feed roller being pulverized in order of first the preceding pulp sheet and then the following pulp sheet,
in the cutting, the trailing end portion of the preceding pulp sheet and the leading end portion of the new following pulp sheet being diagonally cut such that a diagonal cut line of the trailing end portion and a diagonal cut line of the leading end portion have the same inclination, and such that Condition 1 shown below is satisfied with respect to the inclination, and
in the joining, the preceding pulp sheet and the following pulp sheet being joined with use of the joint material in a state where the diagonal cut line of the trailing end portion and the diagonal cut line of the leading end portion have been brought together,
Condition 1: the length of the diagonal cut lines in the feeding direction > a shortest distance from a support position, at which the feed roller supports the pulp sheet, to a movement locus of blade tips of the pulverizing blades.

According to this method for manufacturing an absorbent body, a pull-in phenomenon can be suppressed, and a decrease in the quality of an absorbent body can be suppressed.

In such a method for manufacturing an absorbent body, desirably wherein the joint material is adhesive tape,
in the joining, the preceding pulp sheet and the following pulp sheet are joined by affixing the adhesive tape along the diagonal cut line of the trailing end portion and the diagonal cut line of the leading end portion in a state where the diagonal cut lines have been brought together, and
in the pulverizing, the adhesive tape is pulverized along with the pulp sheet.

According to this method for manufacturing an absorbent body, it is possible to suppress the case where the adhesive tape becomes concentrated in a portion of an absorbent body, and to further suppress a decrease in the quality of the absorbent body.

In such a method for manufacturing an absorbent body, it is desirable that the pulp sheet is unwound from a sheet roll that includes the pulp sheet wound around a paper tube, and an adhesion region for adhesion to the paper tube is provided at a terminal end of the pulp sheet, and
in the cutting, when the trailing end portion of the preceding pulp sheet is cut, cutting is performed at a position ahead of the adhesion region.

According to this method for manufacturing an absorbent body, it is possible to reduce the possibility that the absorption performance of the absorbent body and the sanitary state of the absorbent article will be impaired, and it is possible to further suppress a decrease in the quality of the absorbent body.

In such a method for manufacturing an absorbent body, it is desirable that wherein the pulp sheet is unwound from a sheet roll that includes the pulp sheet wound around a paper tube, and an adhesion region for adhesion to the paper tube is provided at a terminal end of the pulp sheet,
in the cutting, the trailing end portion of the preceding pulp sheet is cut after the pulp sheet has been unwound from the sheet roll and the trailing end portion has separated from the paper tube, and
a regulation member is configured to correct curling of the trailing end portion of the pulp sheet after separating from the paper tube, and the regulation member keeps the trailing end portion of the pulp sheet in a straight linear shape.

According to this method for manufacturing an absorbent body, it is possible to suppress the occurrence of a problem caused by curling of the pulp sheet.

In such a method for manufacturing an absorbent body, it is desirable that wherein an upper surface and a lower surface of the pulp sheet have different fiber densities, and
in the joining, the preceding pulp sheet and the following pulp sheet are joined such that a higher fiber density surface of the preceding pulp sheet and a higher fiber density surface of the following pulp sheet are continuous on one surface of the produced jointed pulp sheet, and such that a lower fiber density surface of the preceding pulp sheet and a lower fiber density surface of the following pulp sheet are continuous on another surface of the produced jointed pulp sheet.

According to this method for manufacturing an absorbent body, it is possible to prevent a rapid change in a production condition for producing fibrous pulp, and to further prevent a decrease in the quality of the absorbent body.

In such a method for manufacturing an absorbent body, it is desirable that wherein the pulp sheet is unwound from a sheet roll that includes the pulp sheet wound around a paper tube, and
the method for manufacturing an absorbent body further comprises attaching to attach the sheet roll to a support portion configured to support the sheet roll, with use of an attaching robot.

According to this method for manufacturing an absorbent body, a sheet roll can be attached to a support portion more quickly than in the case where this operation is performed manually.

An apparatus for manufacturing an absorbent body including:
a pulverizer that includes a plurality of movable pulverizing blades, and is configured to produce a fibrous pulp that is to serve as a material of an absorbent body, by pulverizing a pulp sheet by successively shredding a leading end of the pulp sheet with use of the plurality of pulverizing blades;
a feed roller configured to feed the pulp sheet toward the pulverizer in a feeding direction while supporting the pulp sheet from one end to another end in a width direction of the pulp sheet;
a cutting device configured to cut a trailing end portion of a preceding pulp sheet and a leading end portion of a new following pulp sheet; and
a joining device configured to produce a jointed pulp sheet by joining the preceding pulp sheet and the following pulp sheet together with use of a joint material,
the pulverizer pulverizing the jointed pulp sheet fed with the feed roller in order of first the preceding pulp sheet and then the following pulp sheet,
the cutting device diagonally cutting the trailing end portion of the preceding pulp sheet and the leading end portion of the new following pulp sheet such that a diagonal cut line of the trailing end portion and a diagonal cut line of the leading end portion have the same inclination, and such that Condition 1 shown below is satisfied with respect to the inclination, and
the joining device joining the preceding pulp sheet and the following pulp sheet with use of the joint material in a state where the diagonal cut line of the trailing end portion and the diagonal cut line of the leading end portion have been brought together,
Condition 1: the length of the diagonal cut lines in the feeding direction > a shortest distance from a support position, at which the feed roller supports the pulp sheet, to a movement locus of blade tips of the pulverizing blades.

This apparatus for manufacturing an absorbent body achieves effects similar to those of the method for manufacturing an absorbent body described above.

### Method for manufacturing an absorbent body of present embodiment

A method for manufacturing an absorbent body of the present embodiment is used with an apparatus 10 for manufacturing an absorbent body for manufacturing an absorbent body. In other words, the method for manufacturing an absorbent body of the present embodiment is for manufacturing an absorbent body.

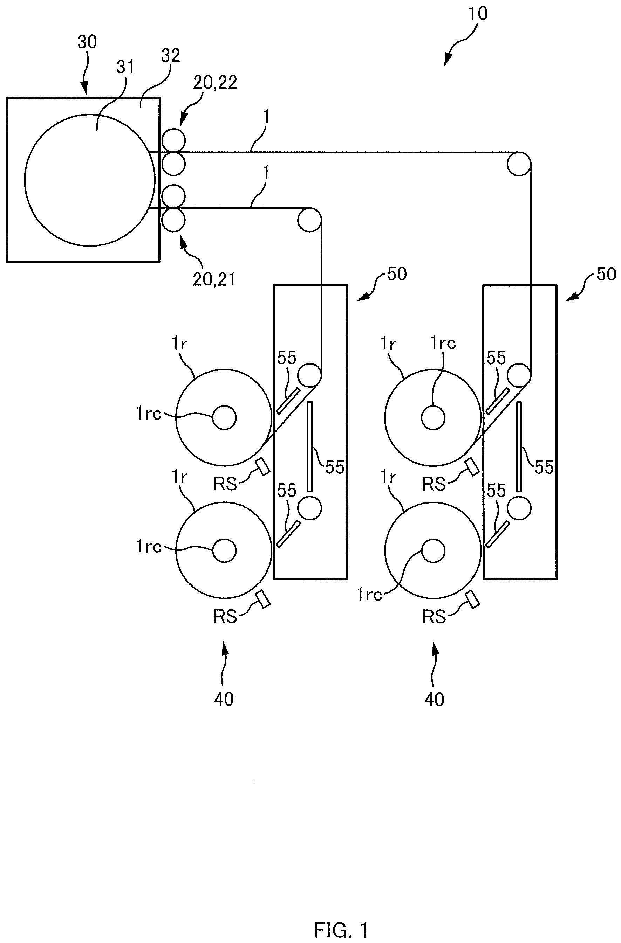

FIG. 1 is a conceptual side view of the apparatus 10 for manufacturing an absorbent body according to the present embodiment. The apparatus 10 for manufacturing an absorbent body (method for manufacturing an absorbent body) of the present embodiment is an apparatus (method) for manufacturing fibrous pulp that is to serve as the material of an absorbent body, by pulverizing a pulp sheet 1 with use of a pulverizer 30.

As shown in FIG. 1, the apparatus 10 for manufacturing an absorbent body of the present embodiment includes: feed rollers 20 that feed the pulp sheet 1 to the pulverizer 30; the pulverizer 30 that pulverizes the fed pulp sheet 1 into fibrous pulp; support portions 40 that each support a sheet roll 1r attached thereto, the sheet rolls 1r each including a pulp sheet 1 that is wound around a paper tube 1rc; and a jointed pulp sheet production device 50 that produces a jointed pulp sheet by joining a preceding pulp sheet 1p and a following pulp sheet 1f.

Also, in the present embodiment, the feeding direction is the longitudinal direction of the pulp sheet 1, and is the direction in which the pulp sheet 1 moves from the support portion 40 toward the pulverizer 30. Accordingly, the width direction of the pulp sheet is a direction passing through the paper plane of FIG. 1. Also, the thickness direction is the direction orthogonal to the feeding direction and the width direction.

Moreover, as shown in FIG. 1, in the apparatus 10 for manufacturing an absorbent body of the present embodiment, the support portions 40, the jointed pulp sheet production device 50, the feed rollers 20, and the pulverizer 30 are arranged in this order in the feeding direction.

### Pulp sheet 1

The pulp sheet 1 of the present embodiment is unwound from the sheet roll 1r, in which the pulp sheet 1 is wound around a paper tube, and the terminal end of the pulp sheet 1 includes an adhesion region J that is adhered to the paper tube 1rc.

Also, the upper surface and the lower surface of the pulp sheet 1 have different fiber densities. This is because of the difference of the finish between the wire surface that was in contact with wires during manufacturing and the opposite surface that was not in contact with such wires. Specifically, the process of producing the pulp sheet 1 from a pulp aqueous dispersion includes a step of disposing the pulp aqueous solution on a wire mesh and allowing the moisture to fall downward through the mesh holes. When the moisture falls downward through the mesh holes in this step, small pulp fibers and the like also fall downward at the same time in the vicinity of the wires. The fiber density is therefore different between the wire surface side and the opposite surface side.

### Pulverizing step

In a pulverizing step of the present embodiment, the feed rollers 20 feed the pulp sheet 1 in the feeding direction toward the pulverizer 30 while supporting the pulp sheet 1 from one end thereof to the other end in the width direction of the pulp sheet, and the pulverizer 30 successively shreds the leading end of the fed pulp sheet 1 with use of multiple moving pulverizing blades 31b, thus producing the fibrous pulp that is to serve as the material of an absorbent body. The feed rollers 20 and the pulverizer 30 will be described below.

### (1) Feed rollers 20

The feed rollers 20 each unwind the pulp sheet 1 from the sheet roll 1r attached to the support portion 40, and feed the pulp sheet 1 toward the pulverizer 30 in the feeding direction while supporting the pulp sheet 1 from one end thereof to the other end in the width direction of the pulp sheet. Here, "supporting the pulp sheet 1 from one end thereof to the other end in the width direction" means that the range supported by the feed roller 20 extends from one end to the other end in the width direction, and it is not necessarily required that the range of the pulp sheet 1 supported by the feed roller 20 is continuous and non-interrupted from the one end to the other end (there may be portions where the feed roller 20 and the pulp sheet 1 are not in contact with each other).

The feed rollers 20 each include a pair of one upper roll and one lower roll, and each has a rotation shaft that extends in the width direction of the pulp sheet 1. At least one of the two rolls is driven to rotate by a servomotor that is the drive source. Also, at least one of the two rolls receives pressing force from a pressing force portion, and the pressing force acts in a direction for bringing the rolls together. Accordingly, the pulp sheet 1 is supported between the roll gap between the upper and lower rolls from one end of the pulp sheet 1 to the other end in the width direction thereof, and the upper and lower rolls are driven to rotate so as to unwind the pulp sheet 1 from the sheet roll 1r and feed the pulp sheet 1 to the pulverizer 30.

Also, as shown in FIG. 1, the feed rollers 20 are provided at positions in the vicinity of the pulverizer 30. This arrangement is employed such that the feed rollers 20 support the pulp sheet 1 at positions near the shredding positions where the pulp sheet 1 is successively shredded by the pulverizing blades 31b of the pulverizer 30. In other words, the leading end of the pulp sheet 1 is the free end that is fed to the pulverizer 30, and the leading end of the pulp sheet 1 is successively shredded and pulverized by the pulverizing blades 31b.

Also, as shown in FIG. 1, the feed rollers 20 of the present embodiment include two feed rollers 20, namely a first feed roller 21 and a second feed roller 22. The first feed roller 21 and the second feed roller 22 are used alternatively. For this reason, the first feed roller 21 and the second feed roller 22 are switched such that while one is feeding the pulp sheet, the other one is stopped.

The feed rollers 20 are switched between the feeding state and the stopped state by a feed roller control unit (not shown) that controls the feeding state of the first feed roller 21 and the second feed roller 22 based on a detection result from a later-described roll remaining amount detection unit RS. Specifically, if there is a small remaining amount of the pulp sheet 1 being fed by one operating feed roller 20, the other stopped feed roller 20 is caused to start feeding the pulp sheet 1, and the one operating feed roller 20 is caused to stop feeding the pulp sheet 1.

Here, the feed roller control unit of the present embodiment performs control such that the one feed roller 20 that is to be stopped does not feed the entirety of the pulp sheet 1 into the pulverizer 30, but rather leaves a portion of the pulp sheet 1 in the later-described jointed pulp sheet production device 50. The remaining portion of the preceding pulp sheet 1p in the jointed pulp sheet production device 50 is joined to a new following pulp sheet 1f to obtain one jointed pulp sheet. In other words, this joining step is performed while feeding is stopped.

Also, the feed roller control unit of the present embodiment performs control to repeatedly switch between the first feed roller 21 and the second feed roller 22 such that the pulp sheet 1 is uninterruptedly fed to the pulverizer 30.

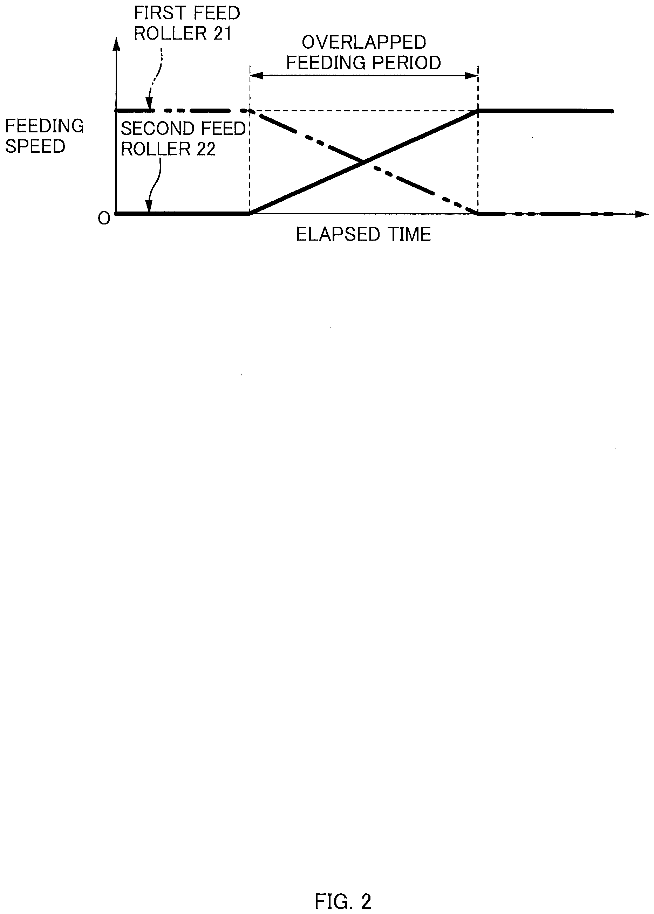

FIG. 2 is a switch timing line chart showing the timing of switching between rollers that feed pulp sheets to the pulverizer 30, specifically switching from the first feed roller 21 that is feeding the pulp sheet 1 to the second feed roller 22 that is stopped. The horizontal axis in the graph in FIG. 2 indicates elapsed time, and the vertical axis indicates the feeding speed.

As shown in FIG. 2, when switching between the feed rollers 20, the feed roller control unit controls the feeding speeds of the first feed roller 21 and the second feed roller 22. Specifically, the feed roller control unit controls the deceleration of the feeding speed of the first feed roller 21 that is to stop feeding and the acceleration of the feeding speed of the second feed roller 22 that is to start feeding, and controls the feeding speeds such that the rate at which the pulp sheet 1 is fed to the pulverizer 30 is the same both when the feed rollers 20 are not being switched (the periods other than the overlapped feeding period in FIG. 2) and when the feed rollers 20 are being switched (overlapped feeding period in FIG. 2). Accordingly, the pulp sheet 1 can be fed to the pulverizer 30 at an appropriate rate regardless of whether or not a switch is performed between the first feed roller 21 and the second feed roller 22.

### (2) Pulverizer 30

The pulverizer 30 includes multiple movable pulverizing blades 31b, and successively shreds the leading end of the pulp sheet 1 with use of the pulverizing blades 31b, thus producing fibrous pulp that is to serve as the material of an absorbent body.

The pulverizer 30 includes a case 32, which is the exterior portion, and a pulverizing portion 31 that has the movable pulverizing blades 31b inside the case 32.

In the present embodiment, the case 32 includes a separate inlet for each of the first feed roller 21 and the second feed roller 22 for introduction of the pulp sheet 1 from the feed rollers 20 into the pulverizer 30.

Also, the pulverizing portion 31 has an approximately circular side surface, and the pulverizing blades 31b are arranged on the outer circumferential portion over a range extending at least from one end of the pulp sheet 1 to the other end in the width direction. The pulverizing portion 31 receives rotation power from an appropriate drive source such as a motor, and is driven to rotate at a constant speed about an axis that extends in the width direction of the pulp sheet 1. Accordingly, when the pulp sheet 1 is fed through the inlet of the case 32, the pulverizing blades 31b pulverize the pulp sheet 1 into fibrous pulp.

### Attaching step

In an attaching step of the present embodiment, an attaching robot (not shown) attaches the sheet rolls 1r to the support portions 40 that are for supporting the sheet rolls 1r.

The support portions 40 are for the attachment and support of the sheet rolls 1r, and in the present embodiment, the support portions 40 are support shafts that extend in the width direction of the pulp sheet 1.

The attaching robot is for attaching the sheet rolls 1r. One example of the attaching robot is a so-called articulated robot that has multiple joints.

Also, in the present embodiment, a roll remaining amount detection unit RS is provided in order to detect the remaining amount of the pulp sheet 1 in the attached sheet roll 1r. One example of the roll remaining amount detection unit RS is a photoelectric tube. The photoelectric tube is arranged between a later-described regulation member 55 and the paper tube 1rc, and detects whether or not the terminal end of the pulp sheet 1 has passed, based on a change in the light reception state when the terminal end of the pulp sheet 1 passes the installation position of the photoelectric tube.

In the present embodiment, as shown in FIG. 1, two support portions 40 are provided for each of the first feed roller 21 and the second feed roller 22, and the support portions 40 all have approximately the same configuration. Accordingly, the first feed roller 21 and the second feed roller 22 each alternatively use one of the two support portions 40, and operate so as to unwind the pulp sheet 1 from the sheet roll 1r that is attached to either one of the support portions 40, and feed the pulp sheet 1 to the pulverizer 30.

In each pair of support portions 40, when the sheet roll 1r attached to one of the support portions 40 is being fed by one of the feed rollers 20, a new following pulp sheet 1f for producing a jointed pulp sheet is attached to the other support portion 40 by the attaching robot (corresponding to the attaching step). The pulp sheets 1 unwound from the two support portions 40 merge in the jointed pulp sheet production device 50, thus producing a jointed pulp sheet.

### Jointed pulp sheet producing step

The jointed pulp sheet producing step includes a cutting step and a joining step.

In the cutting step of the present embodiment, the trailing end portion of a preceding pulp sheet 1p and the leading end portion of a new following pulp sheet 1f are cut diagonally such that the diagonal cut line of the trailing end portion (hereinafter, also called the trailing end portion cut line CLp) and the diagonal cut line of the leading end portion (hereinafter, also called the leading end portion cut line CLf) have the same inclination, and such that, with respect to the inclination, a length La of the trailing end portion cut line CLp and the leading end portion cut line CLf in the feeding direction is longer than a shortest distance Lb from a support position 20a, at which the feed roller 20 supports the pulp sheet 1, to the movement locus of the blade tips of the pulverizing blades 31b.

Also, in the joining step of the present embodiment, a jointed pulp sheet is produced by joining the preceding pulp sheet 1p and the following pulp sheet 1f, which were cut by the cutting device 53, with use of adhesive tape 2 in a state where the trailing end portion cut line CLp of the preceding pulp sheet 1p and the leading end portion cut line CLf of the following pulp sheet 1f have been brought together.

### (1) Jointed pulp sheet production device 50

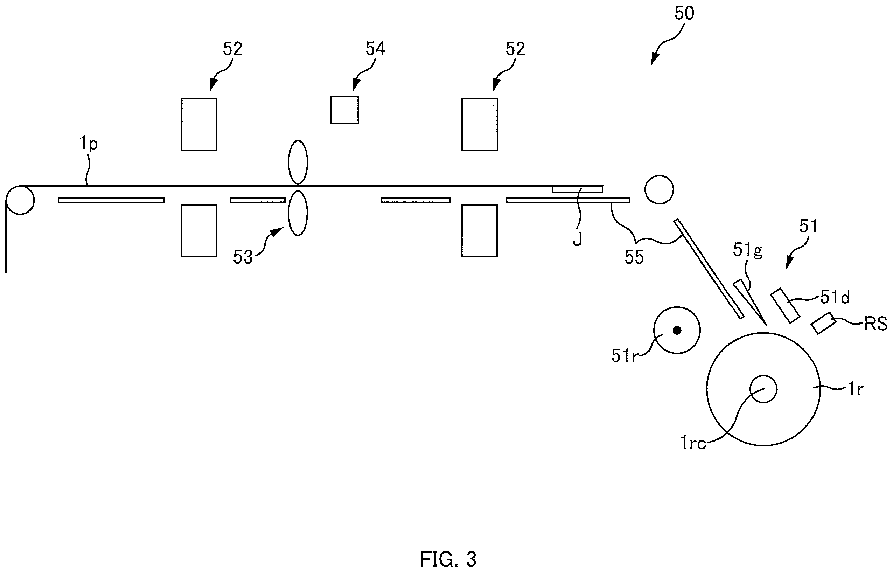

FIG. 3 is a conceptual side view of the jointed pulp sheet production device 50. Note that for the sake of convenience, only one of the two support portions 40 is shown in FIG. 3. The jointed pulp sheet production device 50 is a device that produces a jointed pulp sheet by joining together the preceding pulp sheet 1p and the new following pulp sheet 1f.

The jointed pulp sheet production device 50 of the present embodiment includes an arranging device 51 that arranges the leading end portion of the new following pulp sheet 1f at a predetermined position in the jointed pulp sheet production device 50, fixing devices 52 that fix the pulp sheets so as to not move when pulp sheet cutting and joining are performed, a cutting device 53 that diagonally cuts the preceding pulp sheet 1p and the following pulp sheet 1f, a joining device 54 that produces a jointed pulp sheet by affixing the adhesive tape 2, and regulation members 55 that correct curling of the trailing end portion of the preceding pulp sheet 1p. The arranging device 51, the fixing devices 52, the cutting device 53, the joining device 54, and the regulation members 55 will be described below.

### (a) Arranging device 51

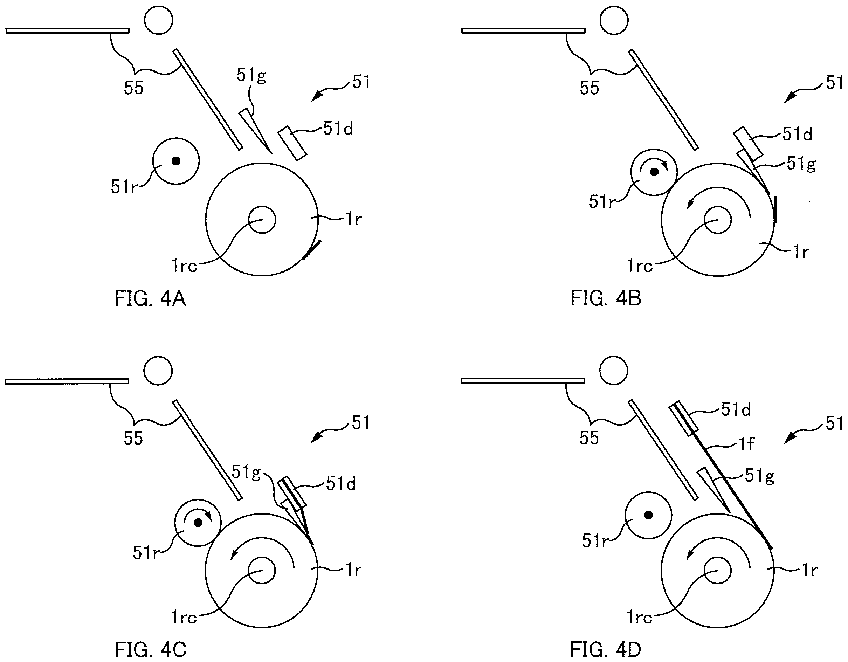

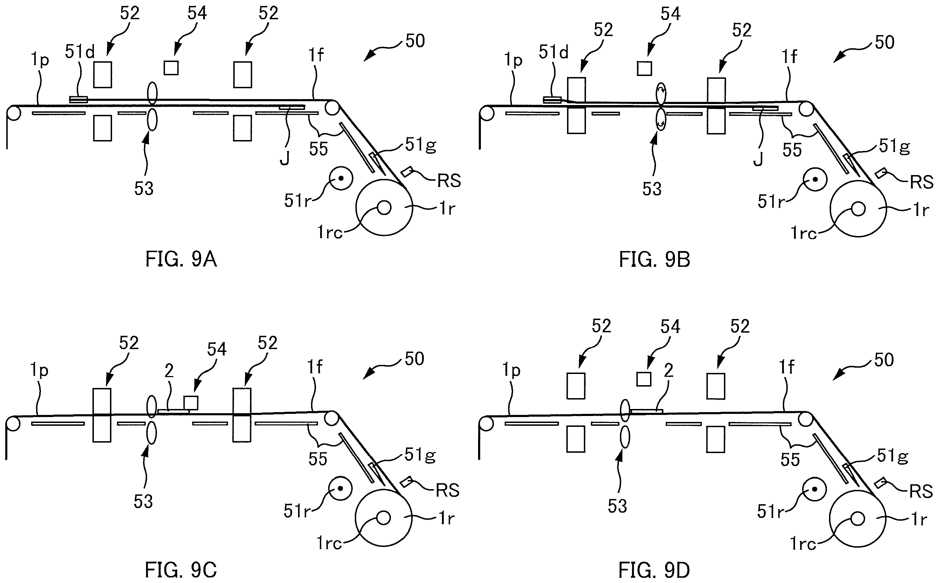

FIGS. 4A to 4D are diagrams showing operations when the arranging device 51 arranges the new following pulp sheet 1f at a predetermined position in the jointed pulp sheet production device 50. Here, the predetermined position is the position of a later-described moving portion 51d shown in FIGS. 9A and 9B, and specifically, is a position at which the leading end portion of the following pulp sheet 1f is located downstream of the downstream fixing device 52 in the feeding direction, and at which the following pulp sheet 1f is processed by the cutting device 53 and the joining device 54 while being fixed by the fixing devices 52.

The arranging device 51 of the present embodiment is a device for arranging the new following pulp sheet 1f at a predetermined position in the jointed pulp sheet production device 50 by operating in the sequence shown in FIGS. 4A, 4B, 4C, and 4D. These operations will be described later.

As shown in FIGS. 4A to 4D, the arranging device 51 of the present embodiment includes the moving portion 51d that moves to a predetermined position in the jointed pulp sheet production device 50 while holding the leading end portion of the following pulp sheet 1f, a guide portion 51g that guides the leading end portion of the following pulp sheet 1f to the moving portion 51d, and a rotation portion 51r that rotates while in contact with the outer circumferential surface of the sheet roll 1r of the following pulp sheet 1f in order to rotate the sheet roll 1r.

### (b) Fixing devices 52

The fixing devices 52 are respectively located on the upstream side and the downstream side of the cutting device 53 and the joining device 54 in the feeding direction, and are devices for fixing the preceding pulp sheet 1p and the following pulp sheet 1f by clamping such pulp sheets from above and below in the thickness direction so as to not move when cutting and joining are performed.

The fixing devices 52 of the present embodiment are each constituted by a pair of clamp portions that have upper and lower side surfaces that are approximately rectangular and extend from one end of the pulp sheets 1 in the width direction to the other end. At least one of the upper and lower clamp portions moves so as to clamp the pulp sheets 1. Also, the fixing devices 52 of the present embodiment fix the preceding pulp sheet 1p and the new following pulp sheet 1f by clamping them at the same time in a state of being overlaid on each other.

### (c) Cutting device 53

The cutting device 53 is a device for diagonally cutting the trailing end portion of the preceding pulp sheet 1p and the leading end portion of the new following pulp sheet 1f such that the trailing end portion cut line CLp and the leading end portion cut line CLf have the same inclination, and such that, with respect to the inclination, the length La of the trailing end portion cut line CLp and the leading end portion cut line CLf in the feeding direction is longer than the shortest distance Lb from the support position 20a, at which the feed roller 20 supports the pulp sheet 1, to the movement locus of the blade tips of the pulverizing blades 31b (corresponding to the cutting step).

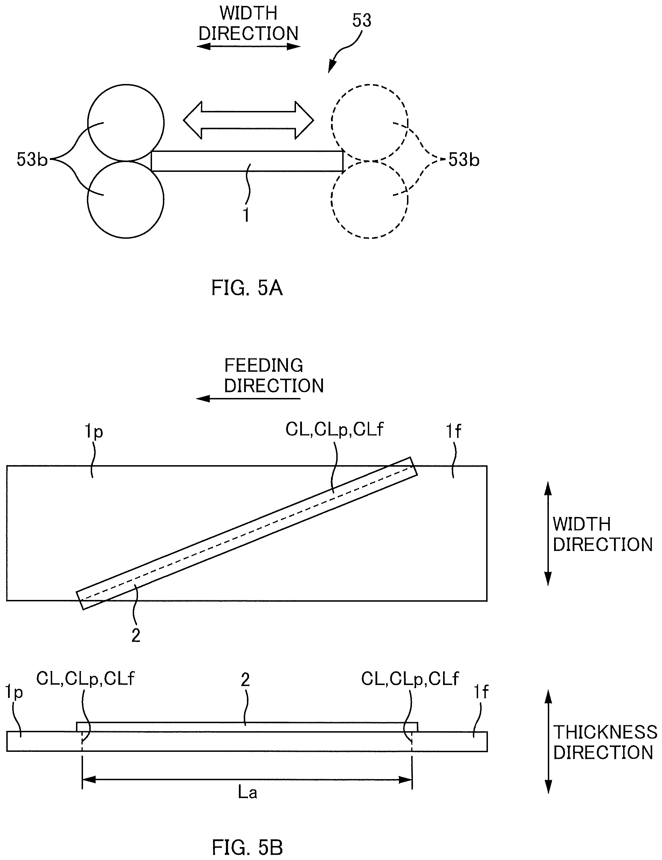

As shown in FIG. 5A, the cutting device 53 of the present embodiment includes a pair of upper and lower rotary shear blades 53b (slitting blades), a rotation drive portion (not shown) that rotates the rotary shear blades 53b, and a drive portion (not shown) that moves the cutting device 53 in order to adjust the cutting direction of the cutting device 53.

FIG. 5B is a conceptual top view and a conceptual side view of a jointed pulp sheet. As shown in FIG. 5B, a joint CL of the jointed pulp sheet is formed as a single joint CL at which the trailing end portion cut line CLp and the leading end portion cut line CLf are brought together. In other words, the cutting device 53 performs cutting such that the trailing end portion cut line CLp and the leading end portion cut line CLf have the same inclination. In the present embodiment, the same inclination is obtained by cutting the preceding pulp sheet 1p and the new following pulp sheet 1f at the same time in a state of being overlaid on each other.

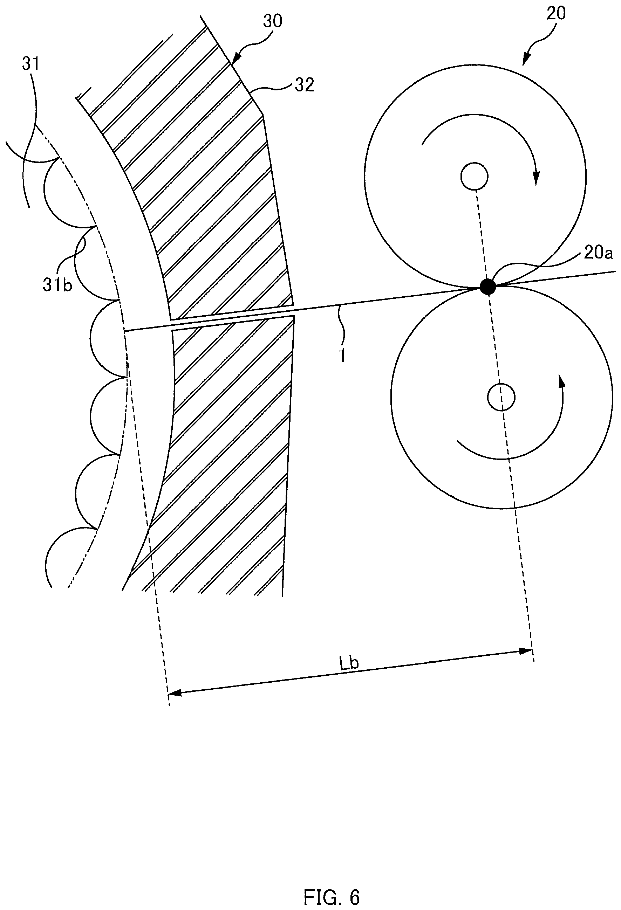

FIG. 6 is an enlarged view that shows the positional relationship between the feed rollers 20 and the pulverizing blades 31b. As previously described, the leading end of the pulp sheet 1 is the free end that is fed to the pulverizer 30, and the leading end of the pulp sheet 1 is successively shredded and pulverized by the pulverizing blades 31b. Also, as shown in FIG. 6, the distance between the support position and the pulverizing position is the shortest distance Lb from the support position 20a, at which the feed rollers 20 support the pulp sheet 1, to the movement locus of the blade tips of the pulverizing blades 31b. Here, as shown in FIG. 5B, the joint CL of the jointed pulp sheet of the present embodiment has the length La in the feeding direction. In the present embodiment, cutting is performed such that the length La is longer than the shortest distance Lb. The reason for this will be described later in the section regarding the effectiveness of the method for manufacturing an absorbent body of the present embodiment.

Also, although the cutting device 53 of the present embodiment includes the pair of upper and lower rotary shear blades 53b, there is no limitation to this. For example, cutting may be performed by pressing a cutter such as a flat-blade knife from above into the preceding pulp sheet 1p and the new following pulp sheet 1f in an overlaid state.

### (d) Joining device 54

The joining device 54 is a device for producing a jointed pulp sheet by joining the preceding pulp sheet 1p and the following pulp sheet 1f, which were cut by the cutting device 53, with use of the adhesive tape 2 in a state where the trailing end portion cut line CLp of the preceding pulp sheet 1p and the leading end portion cut line CLf of the following pulp sheet 1f have been brought together (corresponding to the joining step).

The joining device 54 of the present embodiment has a drive portion that moves the joining device 54 in order to adjust the tape affixing direction of the joining device 54, a tape attaching portion that attaches a tape roll constituted by a roll of the adhesive tape 2, a guide roller that guides a portion of the adhesive tape 2 drawn from the tape roll toward a tape affixing portion, the tape affixing portion that affixes the adhesive tape 2 by pressing the adhesive tape 2, and a tape cutting portion that cuts the adhesive tape 2.

Here, the adhesive tape 2 of the present embodiment is pulverized together with the pulp sheet 1 as part of the jointed pulp sheet, and constitutes part of the fibrous pulp. Accordingly, it is necessary to use a base material and an adhesive that will not impair the absorption performance or the sanitary state of the absorbent article even if included in the absorbent body. Examples include paper adhesive tape, cloth adhesive tape, and film adhesive tape.

### (e) Regulation members 55

After the adhesion region J of the preceding pulp sheet 1p has separated from the paper tube 1rc and been cut, the regulation members 55 correct curling of the trailing end portion of the preceding pulp sheet 1p in order to keep the trailing end portion of the preceding pulp sheet 1p in a straight linear shape.

The regulation members 55 of the present embodiment are approximately plate-shaped members that extend along the conveying route of the pulp sheet 1 over a range from the support portions 40 to the upstream fixing device 52 in the feeding direction. The regulation members 55 may be stainless steel plates, for example.

Also, the regulation members 55 are provided on the side toward which the terminal end of the preceding pulp sheet 1p moves when attempting to return from the straight linear shape to a circular shape due to curling of the trailing end portion of the preceding pulp sheet 1p. In other words, the regulation members 55 are provided on one surface side of the pulp sheet 1 (the side toward which the terminal end moves when attempting to return from the straight linear shape to a circular shape due to curling) along the conveying route of the pulp sheet 1 over a range from the support portions 40 to the upstream fixing device 52 in the feeding direction.

### (2) Jointed pulp sheet production method

The jointed pulp sheet production method of the present embodiment is used with the jointed pulp sheet production device 50. In other words, the jointed pulp sheet production method of the present embodiment is used in the production of the jointed pulp sheet.

The jointed pulp sheet production method will be described below based on operations of the jointed pulp sheet production device 50, with reference to FIGS. 4A to 4D, FIG. 8, and FIGS. 9A to 9D.

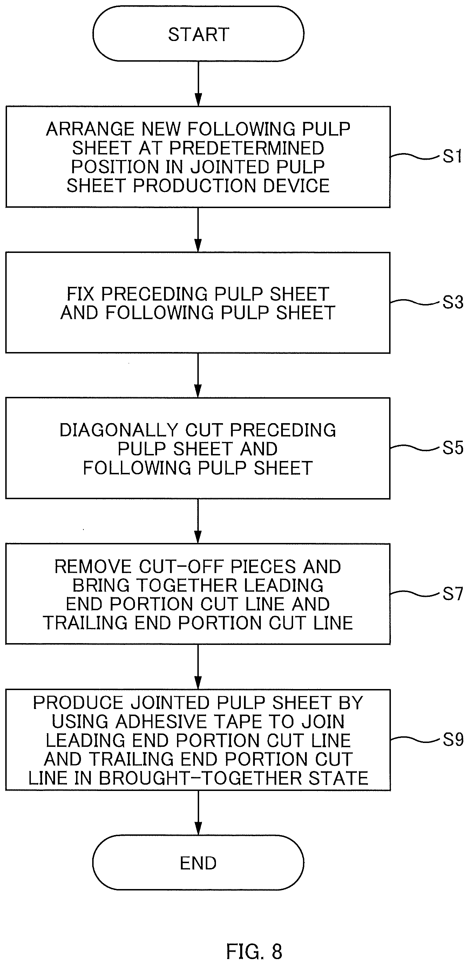

FIG. 8 is a flowchart showing jointed pulp sheet production control. Also, FIGS. 9A to 9D are diagrams showing operations through which the jointed pulp sheet production device 50 produces a jointed pulp sheet. Note that for the sake of convenience, only one of the two support portions 40 is shown in FIGS. 9A to 9D. The following describes the jointed pulp sheet production method of the present embodiment with reference to FIGS. 4A to 4D and FIGS. 9A to 9D, following the flow of the flowchart in FIG. 8.

First, as shown in FIG. 9A, the new following pulp sheet 1f is arranged at a predetermined position in the jointed pulp sheet production device 50 (step S1). Operations of the arranging device 51 of the present embodiment will be described below with reference to FIGS. 4A to 4D.

First, the guide portion 51g and the rotation portion 51r, which are at positions away from the outer circumferential surface of the sheet roll 1r as shown in FIG. 4A, move to positions in contact with the outer circumferential surface of the sheet roll 1r as shown in FIG. 4B. The guide portion 51g has approximately right triangular side surfaces, and the shape extends from one end to the other end in the width direction. The guide portion 51g moves to a position at which the bottom surface of the approximately right triangular shape extends along a tangent of the sheet roll 1r, and furthermore the vertex of the bottom surface and the inclined surface is in contact with the sheet roll 1r. The rotation portion 51r is an approximately circular roller, and the rotation portion 51r moves to a position at which a tangent of the roller extends along a tangent of the sheet roll 1r. The rotation portion 51r is pressed against the sheet roll 1r at this position. The rotation portion 51r includes a rotation shaft that extends in the width direction, a servomotor that drives the rotation shaft to rotate, and a high-friction member on the roller outer circumferential surface that has a high coefficient of friction with respect to the pulp. One example of the high-friction member is a rubber roller. When the rotation portion 51r is driven to rotate in this state, the sheet roll 1r is caused to rotate along with the rotation portion 51r due to friction between the rubber and the pulp that are pressed against each other. In the present embodiment, the rotation portion 51r rotates in the clockwise direction, and the sheet roll 1r rotates in the counter-clockwise direction (the state in FIG. 4B).

Next, when the sheet roll 1r rotates in the state in FIG. 4B, the leading end portion of the sheet roll 1r moves to the position of the guide portion 51g. Here, the leading end portion is not bound to the sheet roll 1r, and therefore is generally separated from the outer circumferential surface of the sheet roll 1r via a gap rather than being in contact therewith. In this state, because the guide portion 51g is in contact with the outer circumferential surface of the sheet roll 1r, the vertex between the bottom surface and the inclined surface of the guide portion 51g enters the gap. In other words, the leading end portion of the sheet roll 1r moves over the inclined surface of the guide portion 51g. The moving portion 51d is provided on the inclined surface side of the guide portion 51g, and the leading end portion moves to the position of the moving portion 51d due to rotation of the sheet roll 1r (the state in FIG. 4C) .

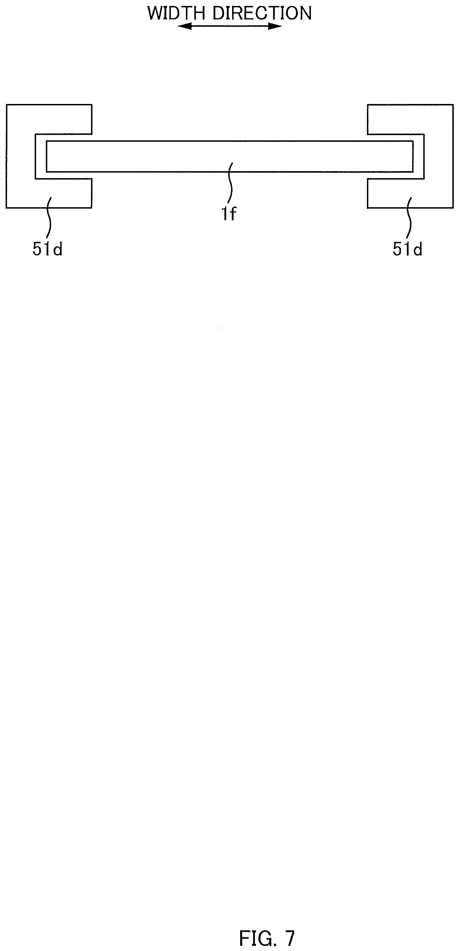

Next, in the state in FIG. 4C, upon reaching the position of the moving portion 51d, the leading end portion of the new following pulp sheet 1f (sheet roll 1r) is held with the moving portion 51d. As shown in FIG. 7, the moving portion 51d of the present embodiment has a configuration in which approximately U-shaped recessed portions face each other. The one end and the other end of the pulp sheet 1 in the width direction are held by being vertically sandwiched with the recessed portions. Specifically, the leading end portion of the new following pulp sheet 1f moves into the approximately U-shaped recessed portions at the one end and the other end in the width direction, and the leading end portion is held due to being vertically sandwiched by the recessed portions. The moving portion 51d includes a drive portion for self-propulsion, and a path guiding portion that guides the path from the position holding the leading end portion to the predetermined position in the jointed pulp sheet production device 50. Accordingly, the moving portion 51d holding the leading end portion moves to the predetermined position by self-propulsion along the path guiding portion.

Although the above-described moving portions 51d are approximately U-shaped and provided as a pair of left and right portions, there is no limitation to this. For example, the moving portions 51d may be approximately rectangular with a gap through which the pulp sheet 1 moves. Also, although the above-described moving portions 51d have a drive portion for self-propulsion, there is no limitation to this. For example, the path guiding portion may have a drive portion that moves back and forth along the path, and the path guiding portion may hold and move the moving portions 51d.

Next, the preceding pulp sheet 1p and the following pulp sheet 1f are fixed (step S3). Specifically, the fixing devices 52 each bring a pair of upper and lower clamp portions toward each other so as to press and fix the preceding pulp sheet 1p and the following pulp sheet 1f in the gap between the pair of upper and lower clamp portions.

Next, as shown in FIG. 9B, the trailing end portion of the preceding pulp sheet 1p and the leading end portion of the new following pulp sheet 1f are cut diagonally such that the trailing end portion cut line CLp and the leading end portion cut line CLf have the same inclination, and such that, with respect to the inclination, the length La of the trailing end portion cut line CLp and the leading end portion cut line CLf in the feeding direction is longer than the shortest distance Lb from the support position 20a, at which the feed roller 20 supports the pulp sheet 1, to the movement locus of the blade tips of the pulverizing blades 31b (step S5, which corresponds to the cutting step). In other words, as shown in FIG. 5A, in the cutting device 53, the pair of upper and lower rotary shear blades 53b move from one end to the other end of the preceding pulp sheet 1p and the following pulp sheet 1f in the width direction thereof while being rotated by the rotation drive portion, thus cutting the preceding pulp sheet 1p and the following pulp sheet 1f. Here, the cutting direction of the cutting device 53 of the present embodiment can be adjusted to any angle between the width direction and the feeding direction. In other words, the cutting device 53 can diagonally cut the trailing end portion of the preceding pulp sheet 1p and the leading end portion of the new following pulp sheet 1f.

Next, the cut-off pieces of the preceding pulp sheet 1p and the following pulp sheet 1f are removed, and the trailing end portion cut line CLp and the leading end portion cut line CLf are brought together (step S7). In the present embodiment, as shown in FIG. 9B, the preceding pulp sheet 1p and the following pulp sheet 1f are cut at the same time in an overlaid state, and therefore the cut-off piece of the preceding pulp sheet 1p is located on one side in the thickness direction of the jointed pulp sheet, and the cut-off piece of the following pulp sheet 1f is located on the other side in the thickness direction. For this reason, a cut-off piece conveying portion (not shown) and a collection container (not shown) for conveying the cut-off piece for removal are provided on both sides in the thickness direction of the jointed pulp sheet. Specifically, the cut-off piece conveying portions provided on both sides convey the cut-off pieces to the corresponding collection containers for removal. In the present embodiment, the preceding pulp sheet 1p and the following pulp sheet 1f are cut at the same time in an overlaid state, and the trailing end portion cut line CLp and the leading end portion cut line CLf are brought together by canceling the fixing of the pulp sheets with the fixing device 52, removing the cut-off pieces, and then again fixing the pulp sheets with the fixing device 52.

Next, as shown in FIG. 9C, a jointed pulp sheet is produced by joining the preceding pulp sheet 1p and the following pulp sheet 1f, which were cut by the cutting device 53, with use of the adhesive tape 2 in a state where the trailing end portion cut line CLp of the preceding pulp sheet 1p and the leading end portion cut line CLf of the following pulp sheet 1f have been brought together (step S9, which corresponds to the joining step). Specifically, while the trailing end portion cut line CLp and the leading end portion cut line CLf have been brought together, the joining device 54 moves the tape affixing portion to a position on one side in the width direction of the trailing end portion cut line CLp and the leading end portion cut line CLf that have been brought together. The tape affixing direction of the joining device 54 is then adjusted so as to extend along the trailing end portion cut line CLp and the leading end portion cut line CLf. The tape affixing portion then presses and affixes the adhesive tape 2 so as to span the boundary between the trailing end portion cut line CLp and the leading end portion cut line CLf. The joining device 54 then moves to the other side in the width direction so as to follow the trailing end portion cut line CLp and the leading end portion cut line CLf while pressing the adhesive tape 2. The adhesive tape 2 is then cut by the tape cutting portion, thus joining the preceding pulp sheet 1p and the following pulp sheet 1f and producing the jointed pulp sheet.

Next, as shown in FIG. 9D, the fixing of the jointed pulp sheet by the fixing device 52 is canceled. The process then waits until the feeding of the jointed pulp sheet to the pulverizer 30 starts.

### Effectiveness of method for manufacturing an absorbent body of present embodiment

As described above, the method for manufacturing an absorbent body of the present embodiment includes: the cutting step in which the trailing end portion of the preceding pulp sheet 1p and the leading end portion of the new following pulp sheet 1f are cut diagonally such that the trailing end portion cut line CLp and the leading end portion cut line CLf have the same inclination, and such that, with respect to the inclination, the length La of the trailing end portion cut line CLp and the leading end portion cut line CLf in the feeding direction is longer than the shortest distance Lb from the support position 20a, at which the feed roller 20 supports the pulp sheet 1, to the movement locus of the blade tips of the pulverizing blades 31b; and the joining step in which a jointed pulp sheet is produced by joining the preceding pulp sheet 1p and the following pulp sheet 1f with use of the adhesive tape 2 in a state where the trailing end portion cut line CLp and the leading end portion cut line CLf have been brought together.

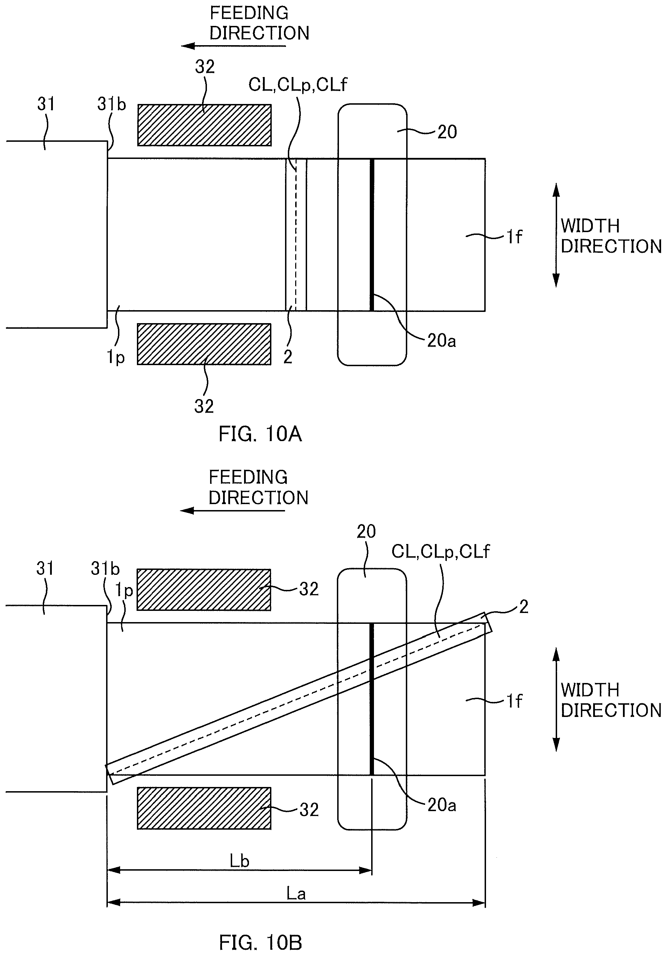

FIG. 10A is a diagram in which a jointed pulp sheet that has been cut along the width direction is being pulverized, and shows a comparative example of the pulverization of an ordinary jointed pulp sheet. FIG. 10B is a diagram in which a jointed pulp sheet that has been cut diagonally is being pulverized, and shows a working example of the pulverization of the jointed pulp sheet according to the present embodiment.

As previously described, the fibrous pulp production method is a method in which a pulp sheet 1 is fed to a pulverizer 30 while being supported at the support position 20a of the feed rollers 20, and the leading end of the fed pulp sheet 1 is shredded and pulverized by the pulverizing blades 31b of the pulverizer 30. For this reason, in the case of the jointed pulp sheet of the comparative example shown in FIG. 10A, after the joint CL has passed the support position 20a of the feed rollers 20, the preceding pulp sheet 1p is supported at the support position 20a of the feed rollers 20 via the adhesive tape 2 and the following pulp sheet 1f. In this situation, it is possible for the adhesive tape 2 to become detached due to pulling force generated by the shredding of the pulverizing blades 31b, and for the preceding pulp sheet 1p to be rapidly pulled into the pulverizer 30 due to the pulling force. In other words, a pull-in phenomenon can occur in which the pulp sheet is pulled into the pulverizer 30.

If this pull-in phenomenon occurs, a large gap is formed between the trailing end of the preceding pulp sheet 1p and the leading end of the following pulp sheet 1f, and the feeding of the pulp sheet 1 into the pulverizer 30 becomes interrupted. Accordingly, there is an increased possibility that an appropriate fibrous pulp cannot be produced by pulverization, an absorbent body having an insufficient amount of fibrous pulp is produced, and the quality of the absorbent body decreases. Also, in this pull-in phenomenon, the pulp sheet 1 is pulled into the pulverizer 30 in a clump-like state, and the pulp sheet 1 is shredded and pulverized in the clump-like state instead of the leading end being shredded and pulverized. In other words, there is an increased possibility that inappropriate fibrous pulp is produced, and that the quality of the absorbent body decreases.

In contrast, in the present embodiment, as shown in FIG. 10B, the trailing end portion of the preceding pulp sheet 1p and the leading end portion of the new following pulp sheet 1f are cut diagonally such that the diagonal cut line of the trailing end portion cut line CLp and the leading end portion cut line CLf have the same inclination, and such that, with respect to the inclination, the length La of the trailing end portion cut line CLp and the leading end portion cut line CLf in the feeding direction is longer than the shortest distance Lb from the support position 20a, at which the feed roller 20 supports the pulp sheet 1, to the movement locus of the blade tips of the pulverizing blades 31b, and a jointed pulp sheet is produced by joining the preceding pulp sheet 1p and the following pulp sheet 1f with use of the adhesive tape 2 in a state where the trailing end portion cut line CLp and the leading end portion cut line CLf have been brought together. Accordingly, as shown in FIG. 10B, even when the joint CL passes the support position 20a of the feed rollers 20, a portion of the joint CL remains rearward of the support position 20a. For this reason, the preceding pulp sheet 1p is supported at the support position 20a of the feed rollers 20 at this time, thus making it possible to suppress the above-described pull-in phenomenon and suppress a decrease in the quality of the absorbent body.

Also, in the joining step of the present embodiment, the preceding pulp sheet 1p and the following pulp sheet 1f are joined by the adhesive tape 2 being affixed thereto along the diagonal cut line in the state where the trailing end portion cut line CLp and the leading end portion cut line CLf have been brought together, and the pulverizer 30 pulverizes the adhesive tape 2 along with the pulp sheets 1.

When the state where the adhesive tape 2 is affixed along the trailing end portion cut line CLp and the leading end portion cut line CLf as shown in FIG. 10B is compared with the state where the adhesive tape 2 is affixed along the width direction as shown in FIG. 10A, it can be seen that a large amount of the adhesive tape 2 is not pulverized at the same time in the state where the adhesive tape 2 is affixed along the trailing end portion cut line CLp and the leading end portion cut line CLf. In other words, it is possible to prevent the adhesive tape 2 that forms a part of the fibrous pulp from becoming concentrated in one portion of the absorbent body, and it is possible to further suppress a decrease in the quality of the absorbent body.

Also, in the cutting step of the present embodiment, when the trailing end portion of the preceding pulp sheet 1p is cut, cutting is performed at a position ahead of the adhesion region J.

The pulp sheet 1 is unwound from the sheet roll 1r that includes the pulp sheet 1 wound around the paper tube 1rc, and the adhesion region J, which is adhered to the paper tube 1rc, is provided at the terminal end of the pulp sheet 1. It is possible for the adhesive that adhered the pulp sheet 1 to the paper tube 1rc and paper waste from the paper tube 1rc upon separation from the paper tube 1rc to adhere to the adhesion region J. Accordingly, when the adhesion region J is fed to the pulverizer 30, the adhesion region J is pulverized and forms a portion of the fibrous pulp, and the aforementioned adhesive and paper waste can possibly be included in the absorbent body. In other words, there is an increased possibility of a decrease in the quality of the absorbent body due to the adhesive and the paper waste impairing the absorption performance and the sanitary state of the absorbent article.

In contrast, in the present embodiment, the adhesion region J, to which adhesive and paper waste from the paper tube 1rc can possibly be attached, is removed beforehand and is not pulverized. In other words, it is possible to reduce the possibility that the absorption performance of the absorbent body and the sanitary state of the absorbent article will be impaired, and it is possible to further suppress a decrease in the quality of the absorbent body.

Also, in the cutting step of the present embodiment, after the pulp sheet 1 is unwound from the sheet roll 1r and has separated from the paper tube 1rc, the trailing end portion of the preceding pulp sheet 1p is cut, and the regulation members 55, which are for correcting curling of the trailing end portion of the pulp sheet 1 after separating from the paper tube 1rc, keep the trailing end portion of the pulp sheet 1 in a straight linear shape.

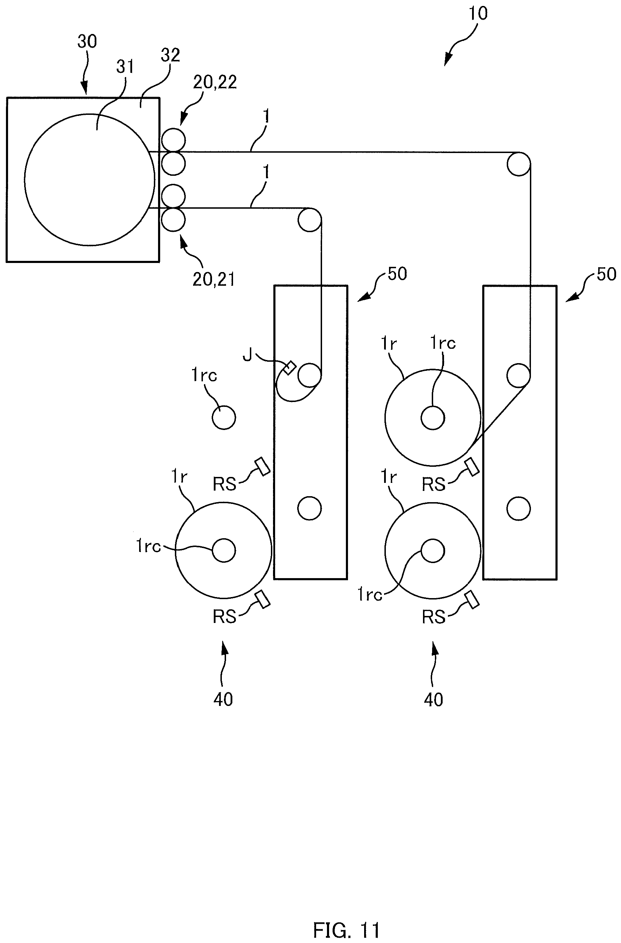

The regulation members 55 correct curling of the trailing end portion of the pulp sheet 1 when the trailing end portion separates from the paper tube 1rc, thus keeping the trailing end portion of the pulp sheet 1 in a straight linear shape, and therefore it is possible to suppress the problem that the trailing end portion rolls up due to curling during the production of the jointed pulp sheet and thus blocks the production of the jointed pulp sheet, for example. Also, as shown in FIG. 11, it is possible to suppress the problem that the pulp sheet 1 wraps around a conveying roller due to curling and thus blocks the feeding thereof to the feed rollers 20.

Also, in the joining step of the present embodiment, the preceding pulp sheet 1p and the following pulp sheet 1f are joined such that the higher fiber density surface of the preceding pulp sheet 1p and the higher fiber density surface of the following pulp sheet 1f are continuous on one surface of the produced jointed pulp sheet, and such that the lower fiber density surface of the preceding pulp sheet 1p and the lower fiber density surface of the following pulp sheet 1f are continuous on the other surface.

This therefore avoids situations where there is rapid increase or decrease in the fiber density of the pulverized surface when the pulverization target changes from the preceding pulp sheet 1p to the following pulp sheet 1f. Accordingly, it is possible to prevent a rapid change in a production condition for producing fibrous pulp, and to further prevent a decrease in the quality of the absorbent body.

Also, in the present embodiment, the production method includes the attaching step in which an attaching robot attaches the sheet rolls 1r to the support portions 40 that are for supporting the sheet rolls 1r.

In other words, the attaching robot can attach the sheet rolls 1r to the support portions 40, and therefore the sheet rolls 1r can be attached to the support portions 40 more quickly than in the case where this operation is performed manually.

### Other embodiments

Although the embodiments of the present disclosure have been described hereinabove, the above embodiments of the present disclosure is simply to facilitate understanding of the present disclosure and are not in any way to be construed as limiting the present disclosure. The present disclosure may variously be changed or altered without departing from its gist and encompass equivalents thereof.

Although two feed rollers 20 are provided for one pulverizer 30 in the embodiment described above, there is no limitation to this. For example, a configuration is possible in which two pulverizers 30 are provided, and one feed roller 20 is provided for each of them.

Also, although two support portions 40 are provided for each feed roller 20 in the above embodiment, there is no limitation to this. For example, one support portion 40 may be provided for one feed roller 20.

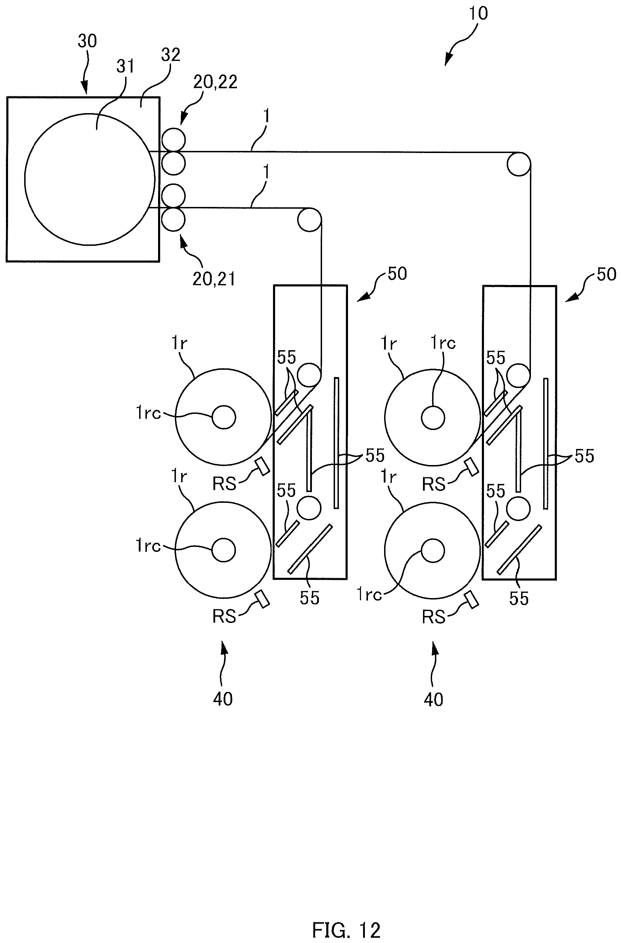

Also, in the above embodiment (first embodiment), the regulation members 55 are provided on only one surface side of the pulp sheet 1 (the side toward which the terminal end moves when attempting to return from the straight linear shape to the circular shape due to curling) along the conveying route of the pulp sheet 1 in a range from the support portions 40 to the downstream fixing device 52 in the feeding direction, but there is no limitation to this. For example, as shown in FIG. 12, the regulation members 55 may be provided on both surface sides of the pulp sheet 1 along the conveying route of the pulp sheet 1 in a range from the support portions 40 to the downstream fixing device 52 in the feeding direction.

The example of the first embodiment is superior in terms of making it possible to reduce the number of regulation members 55. On the other hand, the example of the second embodiment is superior in terms of having an improved effect of correcting curling.

Also, the jointed pulp sheet is produced using the joining device 54 in the above embodiments, and one specific example of the joining device 54 is the general-purpose tape affixing unit MG-2201TU (Marugo Industries Inc.). Note that there is no limitation to this.

Also, although a drive source is not provided for the shafts of the support portions 40 in the above embodiments, there is no limitation to this. For example, a configuration is possible in which a servomotor is provided as a drive source, and the pulp sheet 1 is unwound from the sheet roll 1r by driving the shaft in synchronization with the feed roller 20, and a configuration is possible in which the leading end portion of the new following pulp sheet 1f is arranged at the predetermined position in the jointed pulp sheet production device 50 without using the arranging device 51.

Also, although the above embodiments describe an example where the pulp sheet 1 is fed by the standalone feed roller 20 serving as one aspect of a "feed roller", there is no limitation to this. Another example aspect of the "feed roller" is an aspect in which the pulp sheet 1 is fed via a belt such as an endless belt that is fed with the feed roller 20.

### [Reference Signs List]

1 pulp sheet, 1f following pulp sheet, 1p preceding pulp sheet,
1r sheet roll, 1rc paper tube, 2 adhesive tape,
10 apparatus for manufacturing an absorbent body,
20 feed roller, 20a support position,
21 first feed roller, 22 second feed roller,
30 pulverizer, 31 pulverizing portion, 31b pulverizing blade, 32 case,
40 support portion,
50 jointed pulp sheet production device,
51 arranging device, 51d moving portion, 51g guide portion, 51r rotation portion,
52 fixing device, 53 cutting device, 53b rotary shear blade,
54 joining device, 55 regulation member,
CL joint, CLf leading end portion cut line, CLp trailing end portion cut line,
J adhesion region,
La length of diagonal cut line in feeding direction,
Lb shortest distance from support position to movement locus of blade tips of pulverizing blades
RS roll remaining amount detection unit

## Claims

1. A method for manufacturing an absorbent body comprising:
pulverizing to produce a fibrous pulp that is to serve as a material of an absorbent body, by using a feed roller (20) to feed a pulp sheet (1) toward a pulverizer (30) in a feeding direction while supporting the pulp sheet from one end to another end in a width direction of the pulp sheet, and pulverizing the fed pulp sheet by successively shredding a leading end of the pulp sheet with use of a plurality of moving pulverizing blades (31b) of the pulverizer (30);
cutting to cut a leading end portion (CLp) of a preceding pulp sheet (1p) and a leading end portion (CLf) of a new following pulp sheet (1p); and
joining to produce a jointed pulp sheet by joining the preceding pulp sheet (1p) and the following pulp sheet (1p) with use of a joint material,
in the pulverizing, the jointed pulp sheet fed with the feed roller (20) being pulverized in order of first the preceding pulp sheet (1p) and then the following pulp sheet (1p),
in the cutting, the leading end portion (CLp) of the preceding pulp sheet (1p) and the leading end portion (CLf) of the new following pulp sheet (1p) being diagonally cut such that a diagonal cut line of the leading end portion (CLp) and a diagonal cut line of the leading end portion (CLf) have the same inclination, and such that Condition 1 shown below is satisfied with respect to the inclination, and
in the joining, the preceding pulp sheet (1p) and the following pulp sheet (1p) being joined with use of the joint material in a state where the diagonal cut line of the leading end portion (CLp) and the diagonal cut line of the leading end portion (CLf) have been brought together,
Condition 1: the length (La) of the diagonal cut lines in the feeding direction > a shortest distance (Lb) from a support position, at which the feed roller (20) supports the pulp sheet (1), to a movement locus of blade tips of the pulverizing blades (31b).

2. The method for manufacturing an absorbent body according to claim 1,
wherein the joint material is adhesive tape,
in the joining, the preceding pulp sheet (1p) and the following pulp sheet (1p) are joined by affixing the adhesive tape along the diagonal cut line of the leading end portion (CLp) and the diagonal cut line of the leading end portion (CLf) in a state where the diagonal cut lines have been brought together, and
in the pulverizing, the adhesive tape is pulverized along with the pulp sheet.

3. The method for manufacturing an absorbent body according to claim 1 or 2,
wherein the pulp sheet is unwound from a sheet roll (1r) that includes the pulp sheet wound around a paper tube, and an adhesion region for adhesion to the paper tube is provided at a terminal end of the pulp sheet, and
in the cutting, when the leading end portion (CLp) of the preceding pulp sheet (1p) is cut, cutting is performed at a position ahead of the adhesion region.

4. The method for manufacturing an absorbent body according to any of claims 1 to 3,
wherein the pulp sheet is unwound from a sheet roll (1r) that includes the pulp sheet wound around a paper tube, and an adhesion region for adhesion to the paper tube is provided at a terminal end of the pulp sheet,
in the cutting, the leading end portion (CLp) of the preceding pulp sheet (1p) is cut after the pulp sheet has been unwound from the sheet roll (1r) and the leading end portion (CLp) has separated from the paper tube, and
a regulation member is configured to correct curling of the leading end portion (CLp) of the pulp sheet after separating from the paper tube, and the regulation member keeps the leading end portion (CLp) of the pulp sheet in a straight linear shape.

5. The method for manufacturing an absorbent body according to any of claims 1 to 4,
wherein an upper surface and a lower surface of the pulp sheet have different fiber densities, and
in the joining, the preceding pulp sheet (1p) and the following pulp sheet (1p) are joined such that a higher fiber density surface of the preceding pulp sheet (1p) and a higher fiber density surface of the following pulp sheet (1p) are continuous on one surface of the produced jointed pulp sheet, and such that a lower fiber density surface of the preceding pulp sheet (1p) and a lower fiber density surface of the following pulp sheet (1p) are continuous on another surface of the produced jointed pulp sheet.

6. The method for manufacturing an absorbent body according to any of claims 1 to 5,
wherein the pulp sheet (1) is unwound from a sheet roll (1r) that includes the pulp sheet wound around a paper tube, and
the method for manufacturing an absorbent body further comprises attaching to attach the sheet roll (1r) to a support portion configured to support the sheet roll (1r), with use of an attaching robot.

7. An apparatus for manufacturing an absorbent body comprising:
a pulverizer (30) that includes a plurality of movable pulverizing blades (31b), and is configured to produce a fibrous pulp that is to serve as a material of an absorbent body, by pulverizing a pulp sheet by successively shredding a leading end of the pulp sheet with use of the plurality of pulverizing blades (31b);
a feed roller (20) configured to feed the pulp sheet toward the pulverizer (30) in a feeding direction while supporting the pulp sheet from one end to another end in a width direction of the pulp sheet;
a cutting device configured to cut a leading end portion (CLp) of a preceding pulp sheet (1p) and a leading end portion (CLf) of a new following pulp sheet (1p); and
a joining device configured to produce a jointed pulp sheet by joining the preceding pulp sheet (1p) and the following pulp sheet (1p) together with use of a joint material, the pulverizer (30) pulverizing the jointed pulp sheet fed with the feed roller (20) in order of first the preceding pulp sheet (1p) and then the following pulp sheet (1p),
the cutting device diagonally cutting the leading end portion (CLp) of the preceding pulp sheet (1p) and the leading end portion (CLf) of the new following pulp sheet (1p) such that a diagonal cut line of the leading end portion (CLp) and a diagonal cut line of the leading end portion (CLf) have the same inclination, and such that Condition 1 shown below is satisfied with respect to the inclination, and
the joining device joining the preceding pulp sheet (1p) and the following pulp sheet (1p) with use of the joint material in a state where the diagonal cut line of the leading end portion (CLp) and the diagonal cut line of the leading end portion (CLf) have been brought together,
Condition 1: the length (La) of the diagonal cut lines in the feeding direction > a shortest distance (Lb) from a support position, at which the feed roller (20) supports the pulp sheet, to a movement locus of blade tips of the pulverizing blades (31b).

## Patentansprüche

1. Verfahren zum Herstellen eines absorbierenden Körpers, umfassend:
Pulverisieren, um einen faserigen Zellstoff zu erzeugen, der als ein Material für einen absorbierenden Körper dienen soll, indem eine Zufuhrwalze (20) verwendet wird, um eine Zellstoffbahn (1) in einer Zuführrichtung einem Pulverisierer (30) zuzuführen und dabei die Zellstoffbahn von einem Ende zu einem anderen Ende in einer Breitenrichtung der Zellstoffbahn zu tragen, und Pulverisieren der zugeführten Zellstoffbahn durch sukzessives Zerkleinern eines vorderen Endes der Zellstoffbahn unter Verwendung einer Vielzahl von sich bewegenden Pulverisierungsklingen (31b) des Pulverisierers (30);
Schneiden, um einen vorderen Endabschnitt (CLp) einer vorangehenden Zellstoffbahn (1p) und einen vorderen Endabschnitt (CLf) einer neuen nachfolgenden Zellstoffbahn (1p) zu schneiden; und
Verbinden, um eine verbundene Zellstoffbahn zu erzeugen, indem die vorangehende Zellstoffbahn (1p) und die nachfolgende Zellstoffbahn (1p) unter Verwendung eines Verbindungsmaterials verbunden werden,
wobei beim Pulverisieren die mit der Zuführwalze (20) zugeführte verbundene Zellstoffbahn in der Reihenfolge beginnend mit der vorangehenden Zellstoffbahn (1p) und dann der nachfolgenden Zellstoffbahn (1p) pulverisiert wird,
wobei beim Schneiden der vordere Endabschnitt (CLp) der vorangehenden Zellstoffbahn (1p) und der vordere Endabschnitt (CLf) der neuen nachfolgenden Zellstoffbahn (1p) diagonal geschnitten werden, derart, dass eine diagonale Schnittlinie des vorderen Endabschnitts (CLp) und eine diagonale Schnittlinie des vorderen Endabschnitts (CLf) die gleiche Neigung aufweisen, und derart, dass die unten gezeigte Bedingung 1 in Bezug auf die Neigung erfüllt ist, und
beim Verbinden die vorangehende Zellstoffbahn (1p) und die nachfolgende Zellstoffbahn (1p) unter Verwendung des Verbindungsmaterials in einem Zustand verbunden werden, in dem die diagonale Schnittlinie des vorderen Endabschnitts (CLp) und die diagonale Schnittlinie des vorderen Endabschnitts (CLf) zusammengeführt wurden,
Bedingung 1: Länge (La) der diagonalen Schnittlinien in Zuführrichtung > kürzester Abstand (Lb) von einer Tragposition, an der die Zuführwalze (20) die Zellstoffbahn (1) trägt, zu einem Bewegungsort von Klingenspitzen der Pulverisierungsklingen (31b).

2. Verfahren zum Herstellen eines absorbierenden Körpers nach Anspruch 1,
wobei das Verbindungsmaterial Klebeband ist,
beim Verbinden die vorangehende Zellstoffbahn (1p) und die nachfolgende Zellstoffbahn (1p) durch Fixieren des Klebebands entlang der diagonalen Schnittlinie des vorderen Endabschnitts (CLp) und der diagonalen Schnittlinie des vorderen Endabschnitts (CLf) in einem Zustand verbunden werden, in dem die diagonalen Schnittlinien zusammengeführt wurden, und
beim Pulverisieren das Klebeband zusammen mit der Zellstoffbahn pulverisiert wird.

3. Verfahren zum Herstellen eines absorbierenden Körpers nach Anspruch 1 oder 2,
wobei die Zellstoffbahn von einer Bahnrolle (1r) abgewickelt wird, die die um eine Papierröhre gewickelte Zellstoffbahn beinhaltet, und an einem Abschlussende der Zellstoffbahn ein Klebebereich zum Anhaften an der Papierröhre bereitgestellt ist, und
beim Schneiden, wenn der vordere Endabschnitt (CLp) der vorangehenden Zellstoffbahn (1p) geschnitten wird, das Schneiden an einer Position vor dem Klebebereich durchgeführt wird.

4. Verfahren zum Herstellen eines absorbierenden Körpers nach einem der Ansprüche 1 bis 3,
wobei die Zellstoffbahn von einer Bahnrolle (1r) abgewickelt wird, die die um eine Papierröhre gewickelte Zellstoffbahn beinhaltet, und an einem Abschlussende der Zellstoffbahn ein Klebebereich zum Anhaften an der Papierröhre bereitgestellt ist,
beim Schneiden der vordere Endabschnitt (CLp) der vorangehenden Zellstoffbahn (1p) abgeschnitten wird, nachdem die Zellstoffbahn von der Bahnrolle (1r) abgewickelt wurde und sich der vordere Endabschnitt (CLp) von der Papierröhre getrennt hat, und
ein Regulierungsglied dazu konfiguriert ist, eine Wellung des vorderen Endabschnitts (CLp) der Zellstoffbahn nach der Trennung von der Papierröhre zu korrigieren, und das Regulierungsglied den vorderen Endabschnitt (CLp) der Zellstoffbahn in einer geraden, linearen Form hält.

5. Verfahren zum Herstellen eines absorbierenden Körpers nach einem der Ansprüche 1 bis 4,
wobei eine obere Fläche und eine untere Fläche der Zellstoffbahn unterschiedliche Faserdichten aufweisen, und
beim Verbinden die vorangehende Zellstoffbahn (1p) und die nachfolgende Zellstoffbahn (1p) derart verbunden werden, dass eine Fläche mit höherer Faserdichte der vorangehenden Zellstoffbahn (1p) und eine Fläche mit höherer Faserdichte der nachfolgenden Zellstoffbahn (1p) auf einer Fläche der erzeugten verbundenen Zellstoffbahn durchgängig sind, und derart, dass eine Fläche mit geringerer Faserdichte der vorangehenden Zellstoffbahn (1p) und eine Fläche mit geringerer Faserdichte der nachfolgenden Zellstoffbahn (1p) auf einer anderen Fläche der hergestellten Zellstoffbahn durchgängig sind.

6. Verfahren zum Herstellen eines absorbierenden Körpers nach einem der Ansprüche 1 bis 5,
wobei die Zellstoffbahn (1) von einer Bahnrolle (1r) abgewickelt wird, die die um eine Papierröhre gewickelte Zellstoffbahn beinhaltet, und
das Verfahren zum Herstellen eines absorbierenden Körpers ferner Befestigen umfasst, um die Bahnrolle (1r) unter Verwendung eines Befestigungsroboters an einem Trägerabschnitt zu befestigen, der zum Tragen der Bahnrolle (1r) konfiguriert ist.

7. Vorrichtung zum Herstellen eines absorbierenden Körpers, umfassend:
einen Pulverisierer (30), der eine Vielzahl von bewegbaren Pulverisierungsklingen (31b) beinhaltet und dazu konfiguriert ist, einen faserigen Zellstoff zu erzeugen, der als ein Material für einen absorbierenden Körper dienen soll, indem eine Zellstoffbahn durch sukzessives Zerkleinern eines vorderen Endes der Zellstoffbahn unter Verwendung der Vielzahl von Pulverisierungsklingen (31b) pulverisiert wird;
eine Zuführwalze (20), die dazu konfiguriert ist, die Zellstoffbahn in einer Zuführrichtung dem Pulverisierer (30) zuzuführen und dabei die Zellstoffbahn von einem Ende zu einem anderen Ende in einer Breitenrichtung der Zellstoffbahn zu tragen;
eine Schneidvorrichtung, die dazu konfiguriert ist, einen vorderen Endabschnitt (CLp) einer vorangehenden Zellstoffbahn (1p) und einen vorderen Endabschnitt (CLf) einer neuen nachfolgenden Zellstoffbahn (1p) zu schneiden; und
eine Verbindungsvorrichtung, die dazu konfiguriert ist, eine verbundene Zellstoffbahn zu erzeugen, indem die vorangehende Zellstoffbahn (1p) und die nachfolgende Zellstoffbahn (1p) unter Verwendung eines Verbindungsmaterials miteinander verbunden werden,
wobei der Pulverisierer (30) die mit der Zuführwalze (20) zugeführte verbundene Zellstoffbahn in der Reihenfolge beginnend mit der vorangehenden Zellstoffbahn (1p) und dann der nachfolgenden Zellstoffbahn (1p) pulverisiert,
wobei die Schneidvorrichtung den vorderen Endabschnitt (CLp) der vorangehenden Zellstoffbahn (1p) und den vorderen Endabschnitt (CLf) der neuen nachfolgenden Zellstoffbahn (1p) derart schneidet, dass eine diagonale Schnittlinie des vorderen Endabschnitts (CLp) und eine diagonale Schnittlinie des vorderen Endabschnitts (CLf) die gleiche Neigung aufweisen, und derart, dass die unten gezeigte Bedingung 1 in Bezug auf die Neigung erfüllt ist, und
die Verbindungsvorrichtung die vorangehende Zellstoffbahn (1p) und die nachfolgende Zellstoffbahn (1p) unter Verwendung des Verbindungsmaterials in einem Zustand verbindet, in dem die diagonale Schnittlinie des vorderen Endabschnitts (CLp) und die diagonale Schnittlinie des vorderen Endabschnitts (CLf) zusammengeführt wurden,
Bedingung 1: Länge (La) der diagonalen Schnittlinien in Zuführrichtung > kürzester Abstand (Lb) von einer Tragposition, an der die Zuführwalze (20) die Zellstoffbahn trägt, zu einem Bewegungsort von Klingenspitzen der Pulverisierungsklingen (31b) .

## Revendications

1. Procédé de fabrication d'un corps absorbant comprenant :
la pulvérisation pour produire une pâte fibreuse qui doit servir de matériau d'un corps absorbant, en utilisant un rouleau d'alimentation (20) pour alimenter une feuille de pâte (1) vers un pulvérisateur (30) dans une direction d'alimentation tout en supportant la feuille de pâte depuis une extrémité à une autre extrémité dans le sens de la largeur de la feuille de pâte, et la pulvérisation de la feuille de pâte alimentée en déchiquetant successivement une extrémité avant de la feuille de pâte à l'aide d'une pluralité de lames de pulvérisation mobiles (31b) du pulvérisateur (30) ;
la découpe pour couper une partie d'extrémité avant (CLp) d'une feuille de pâte précédente (1p) et une partie d'extrémité avant (CLf) d'une nouvelle feuille de pâte suivante (1p) ; et
l'assemblage pour produire une feuille de pâte jointe en joignant la feuille de pâte précédente (1p) et la feuille de pâte suivante (1p) à l'aide d'un matériau d'assemblage,
lors de la pulvérisation, la feuille de pâte jointe alimentée par le rouleau d'alimentation (20) étant pulvérisée dans l'ordre d'abord la feuille de pâte précédente (1p) puis la feuille de pâte suivante (1p),
lors de la découpe, la partie d'extrémité avant (CLp) de la feuille de pâte précédente (1p) et la partie d'extrémité avant (CLf) de la nouvelle feuille de pâte suivante (1p) étant coupées en diagonale de sorte qu'une ligne de coupe diagonale de la partie d'extrémité avant (CLp) et une ligne de coupe diagonale de la partie d'extrémité avant (CLf) ont la même inclinaison, et de sorte que la condition 1 indiquée ci-dessous est satisfaite en ce qui concerne l'inclinaison, et
lors de l'assemblage, la feuille de pâte précédente (1p) et la feuille de pâte suivante (1p) étant jointes à l'aide du matériau d'assemblage dans un état où la ligne de coupe diagonale de la partie d'extrémité avant (CLp) et la ligne de coupe diagonale de la partie d'extrémité avant (CLf) ont été réunis,
Condition 1 : la longueur (La) des lignes de coupe diagonales dans la direction d'alimentation > une distance la plus courte (Lb) depuis une position de support, à laquelle le rouleau d'alimentation (20) supporte la feuille de pâte (1), jusqu'à un lieu de déplacement de pointes de lames des lames de pulvérisation (31b).

2. Procédé de fabrication d'un corps absorbant selon la revendication 1,
dans lequel le matériau d'assemblage est un ruban adhésif,
lors de l'assemblage, la feuille de pâte précédente (1p) et la feuille de pâte suivante (1p) sont jointes en fixant le ruban adhésif le long de la ligne de coupe diagonale de la partie d'extrémité avant (CLp) et de la ligne de coupe diagonale de la partie d'extrémité avant (CLf) dans un état où les lignes de coupe diagonales ont été réunis, et
lors de la pulvérisation, le ruban adhésif est pulvérisé avec la feuille de pâte.

3. Procédé de fabrication d'un corps absorbant selon la revendication 1 ou 2,
dans lequel la feuille de pâte est déroulée d'un rouleau de feuille (1r) qui comporte la feuille de pâte enroulée autour d'un tube de papier, et une région d'adhérence pour l'adhésion au tube de papier est prévue à une extrémité terminale de la feuille de pâte, et
lors de la découpe, lorsque la partie d'extrémité avant (CLp) de la feuille de pâte précédente (1p) est coupée, la découpe est effectuée à une position en avant de la région d'adhérence.

4. Procédé de fabrication d'un corps absorbant selon l'une quelconque des revendications 1 à 3,
dans lequel la feuille de pâte est déroulée d'un rouleau de feuille (1r) qui comporte la feuille de pâte enroulée autour d'un tube de papier, et une région d'adhérence pour l'adhésion au tube de papier est prévue à une extrémité terminale de la feuille de pâte,
lors de la découpe, la partie d'extrémité avant (CLp) de la feuille de pâte précédente (1p) est coupée après que la feuille de pâte a été déroulée du rouleau de feuille (1r) et que la partie d'extrémité avant (CLp) s'est séparée du tube de papier, et
un élément de régulation est configuré pour corriger le gondolage de la partie d'extrémité avant (CLp) de la feuille de pâte après séparation du tube de papier, et l'élément de régulation maintient la partie d'extrémité avant (CLp) de la feuille de pâte dans une forme linéaire droite.

5. Procédé de fabrication d'un corps absorbant selon l'une quelconque des revendications 1 à 4,
dans lequel une surface supérieure et une surface inférieure de la feuille de pâte ont des densités de fibres différentes, et
lors de l'assemblage, la feuille de pâte précédente (1p) et la feuille de pâte suivante (1p) sont jointes de sorte qu'une surface à densité de fibres plus élevée de la feuille de pâte précédente (1p) et une surface à densité de fibres plus élevée de la feuille de pâte suivante (1p) sont continues sur une surface de la feuille de pâte jointe produite, et telles qu'une surface à plus faible densité de fibres de la feuille de pâte précédente (1p) et une surface à plus faible densité de fibres de la feuille de pâte suivante (1p) sont continues sur une autre surface de la feuille de pâte jointe produite.

6. Procédé de fabrication d'un corps absorbant selon l'une quelconque des revendications 1 à 5,
dans lequel la feuille de pâte (1) est déroulée d'un rouleau de feuille (1r) qui comporte la feuille de pâte enroulée autour d'un tube de papier, et
le procédé de fabrication d'un corps absorbant comprend en outre la fixation pour fixer le rouleau de feuille (1r) à une partie de support configurée pour supporter le rouleau de feuille (1r), à l'aide d'un robot de fixation.

7. Appareil de fabrication d'un corps absorbant comprenant :
un pulvérisateur (30) qui comporte une pluralité de lames de pulvérisation mobiles (31b), et est configuré pour produire une pâte fibreuse qui doit servir de matériau d'un corps absorbant, en pulvérisant une feuille de pâte en déchiquetant successivement une extrémité avant de la feuille de pâte avec utilisation de la pluralité de lames de pulvérisation (31b) ;
un rouleau d'alimentation (20) configuré pour alimenter la feuille de pâte vers le pulvérisateur (30) dans une direction d'alimentation tout en supportant la feuille de pâte d'une extrémité à une autre extrémité dans le sens de la largeur de la feuille de pâte ;
un dispositif de découpe configuré pour couper une partie d'extrémité avant (CLp) d'une feuille de pâte précédente (1p) et une partie d'extrémité avant (CLf) d'une nouvelle feuille de pâte suivante (1p) ; et
un dispositif d'assemblage pour produire une feuille de pâte jointe en joignant la feuille de pâte précédente (1p) et la feuille de pâte suivante (1p) à l'aide d'un matériau d'assemblage,
le pulvérisateur (30) pulvérisant la feuille de pâte jointe alimentée par le rouleau d'alimentation (20) dans l'ordre d'abord la feuille de pâte précédente (1p) puis la feuille de pâte suivante (1p),
le dispositif de découpe coupant diagonalement la partie d'extrémité avant (CLp) de la feuille de pâte précédente (1p) et la partie d'extrémité avant (CLf) de la nouvelle feuille de pâte suivante (1p) de sorte qu'une ligne de coupe diagonale de la partie d'extrémité avant (CLp) et une ligne de coupe diagonale de la partie d'extrémité avant (CLf) ont la même inclinaison, et de sorte que la condition 1 indiquée ci-dessous est satisfaite en ce qui concerne l'inclinaison, et
le dispositif d'assemblage joignant la feuille de pâte précédente (1p) et la feuille de pâte suivante (1p) à l'aide du matériau d'assemblage dans un état où la ligne de coupe diagonale de la partie d'extrémité avant (CLp) et la ligne de coupe diagonale de la partie d'extrémité avant (CLf) ont été réunis,
Condition 1 : la longueur (La) des lignes de coupe diagonales dans la direction d'alimentation > une distance la plus courte (Lb) depuis une position de support, à laquelle le rouleau d'alimentation (20) supporte la feuille de pâte, jusqu'à un lieu de déplacement de pointes de lames des lames de pulvérisation (31b).
